Europäisches Patentamt

European Patent Office

Office européen des brevets

(11) Publication number: **0 298 533**
**A1**

## (12) EUROPEAN PATENT APPLICATION

(21) Application number: **88201141.4**

(22) Date of filing: **06.06.88**

(51) Int. Cl.⁴ **A61L 25/00 , A61L 27/00 , A61L 31/00**

(30) Priority: **12.06.87 NL 8701370**

(43) Date of publication of application:
**11.01.89 Bulletin 89/02**

(84) Designated Contracting States:
**AT BE CH DE ES FR GB GR IT LI LU NL SE**

(71) Applicant: **SURGICAL RESEARCH FOUNDATION**
**Amstel 65**
**NL-1018 EJ Amsterdam(NL)**

(72) Inventor: **Klopper Pieter Jacobus**
**Amstel 65**
**NL-1018 EJ Amsterdam(NL)**
Inventor: **Meijer Dirk**
**Pontanusstraat 9**
**NL-1093 RW Amsterdam(NL)**

(74) Representative: **Flamman, Han**
**LIOC Patents and Licensing P.O. Box 54**
**NL-3970 AB Driebergen(NL)**

(54) **Sheep dermal collagen as an adhesive medium for living tissues.**

(57) Foil of tanned sheep dermal collagen, to be used as a fast working medium for uniting living tissue or parts thereof to other (parts of) living tissue.

For instance for fixing outer surfaces of walls of organs with respect to one another; for uniting parts of organs with each other and for fixing organs in a predetermined position.

For instance also for protecting sutures and as a bone-cement.

EP 0 298 533 A1

Fig. 2

# SHEEP DERMAL-COLLAGEN AS A MEDIUM FOR UNITING LIVING TISSUES.

The invention relates to a foil of tanned, sheep dermal-collagen commonlyreferred to a 'shammy leather':

In Dr. Van Gulik's thesis "Processed sheep dermal-collagen as a biomaterial", some of the properties of the material were described therein such as its biological decomposability; when introduced into a living organism it induces the formation of the organs own collagen and exhibits only a small degree of reaction to foreign-bodies.

As medical applications hereof, its use as a wound-covering material and as an implant in the reconstruction of blood vessels may be quoted.

It appears that the quoted sheep's collagen unites very quickly with living tissue, that is to say within a few seconds and the present invention rests on the insight that these properties of the collagen can be used for various medical applications when made into the form of a adhesive/uniting medium for living tissues. After the tissues have thus united with one another the collagen is 'invaded' by the 'host' matter and within a few days a good growth exists therebetween.

In this respect, Dr. Gulik's paper titled "'Lower split-skin sheep's dermal-collagen" described in the quoted publication particularly satisfies this theory, that is to say that after longitudinal unison a single entity of skin is obtained and that with the aid of a glutinous aldehyde a so-called "High-grade tanning process" is undergone. Consequently the invention is characterised in that the said collagen is used as a fast-working unitingve medium for causing one living tissue or part thereof to unite with another living tissue or part thereof respectively. Use is then made of the uniting properties of the collagen and in which in addition to the properties quoted in the foregoingly quoted thesis these properties work more favourably.

A first specific application is in introducing a valve into an organ having a transporting function such as the intestines, gall or urinary tracts.

It frequently occurs that surgery must be performed to provide such an organ with means for preventing the flow of the contents in two directions. For example, this occurs when a human valve-mechanism is surgically removed or has become blocked. In such cases the flow needs to be limited to one direction only and to this end a 'valve' is constructed in the organ concerned. A known method of achieving this is through so-called 'invagination, a process in which a part of the organ's wall is circumferetially doubled-back on another part by pressing together adjacent parts of the wall in the longitudinal direction and in which a kind of sleeve is formed thereover and through which there is provided a construction operating like a valve.

In order to fix an invagination-formed valve in its invaginated condition, use is usually made of a lightweight material in the form of stainless-steel staples. Through movement of the intestines for example, such a fixing method is insufficiently resistant to such movement and in many cases the fixing media again becomes undone. This means that in such cases surgery must be resorted to for a second time. This occurs with 30% of intestinal operations.

The invention now incorporates the use of the collagen foils in achieving the hereinbefore quoted fixation of a vagination, that is to say by means of a sleeve formed thereby which is arranged around the exterior surface of that part of the organ concerned lyig thereunder. This application is thus characterised in that such a foil is used to fix adjacently lying surfaces of the wall of a transporting organ with respect to one another by the introduction of the said foil around and between both the said surfaces of the organ into which a valve has been invaginated.

The good results obtained herefrom can be illustrated as follows:
Operations were performed on eight dogs in which a valve was constructed in an intestine by invagination. Fixation was carried out using the known method with staples. In five cases the staples-suture broke loose.

In addition hereto, thirty dogs underwent the same operation with use made of sheep dermal-collagen as the adhesive/uniting medium. In no single case did this form of suture break loose.
The foil to be used for the forgoingly quoted purpose is preferably preformed and well in the sense that it is mainly of rectangular form and of which two parallel sides have approximately the same length as the circumferential cross-section of the organ concerned whilst, when viewed in one direction, the foil is extended along one side with a lip and on the other side with a strip the length of which is approximately equal to that of an extension of the said side.

In applying the collagen around the organ, the said lip must be pushed into the so-called misentery whilst the long strip encompasses the mesentery on the upper side of the invaginate. The mesentery is a fleshlike tissue by which means an intestine is affixed in place on the rear wall of the abdomen, it includes the blood vessels leading to the intestines themselves.

In addition to the hereinbefore described application of the collagen foil in fixing invaginates, it has just as much appeared that extremely favourable results have been achieved when used:
- to unite severed hard cerebral membrane together after after a brain operation;
- as a means for uniting severed parts of a transporting organ such as ureter parts.
- for fixing kidneys or a gall organ to the rear wall of the abdomen;
- for uniting ruptures between parts of the abdomen wall; and for filling up abdomen wall defects arising when the wall is subjected to certain tension;
as a means of uniting separated tendons to one another;
- as a means of protecting sutures when the concerned walls are not in good condition;
- for uniting joints parts together;
- as an artificial ligament;
- as a bone-uniting cement.

In the various uses of collagen foils, it is often advantageous to treat the foils with antibiotics, antiseptics, blood anti-staunching media, cell stimulants or possibly combinations of these.

The invention is now to be described with reference to the accompanying drawings and with respect to the manner in which collagen foils according to the invention are used in invaginations for fixing a tubular transporting organ such as an intestine.

Figures 1 and 2 showing longitudinal cross-sections of an intestine also shows in principle how an invagination is fixed in place.

Figure 3 also shows a longitudinal cross-section of an intestine and how an intestinal invagination fixed in accordance with the invention looks in practice and in which there is shown how the mesentery comes to lie in such a case;

Figure 4 shows a preformed foil according to the invention.

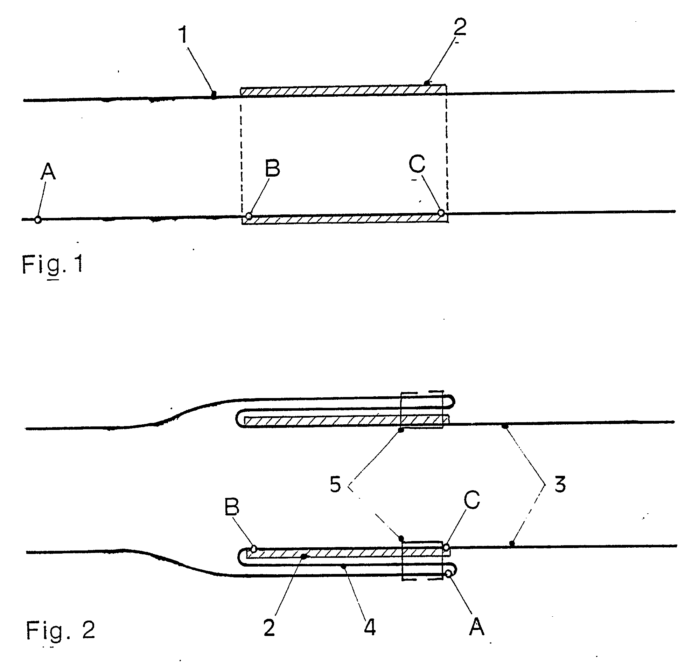

Referring now particularly to fig.1, this shows a cross-section of a tubular organ which is to be provided with a valve between portions B and C and which is ensheathed over a length therebetween with a foil 2. It is to be commented here that the ensheathment over only a part of the length under certain circumstances leads to sufficient unison. Invagination then takes place to produce a situation as shown in fig. 2 which is also in cross-section.

The point on the surface indicated by the reference numeral A in fig. 1 is in the invaginated condition with respect to point C, see fig. 2. The foil then lies between both the surfaces 3 and 4 of the invaginate. At points A and C approximately, and thus on the upper side of the invagiate, the invaginate itself is circumferentially affixed with the aid of sutures 5.

After some days, tissue growth has formed between the sheep dermal-collagen foil 2 and the surface 3 on one side and and the sheep dermal-collagen foil 2 and the surface 4 on the other side and through which the invagination remains fixed.

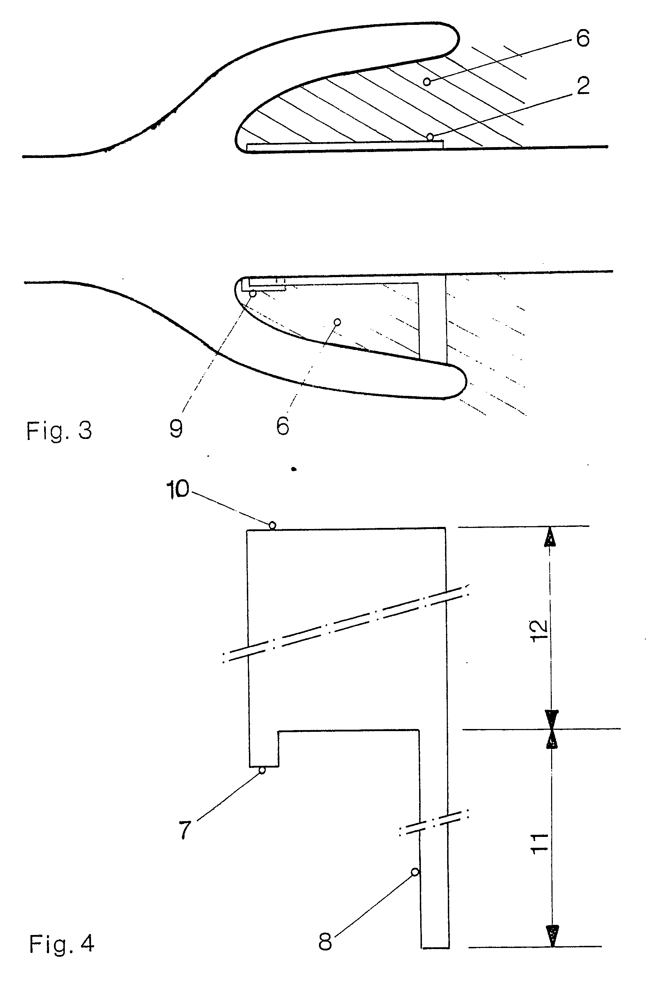

Figure 3 shows the invagination as shown in principle in fig. 2 and in which there is shown how the foil 2 is applied when intestines are dealt with in practice when they are united with the abdomen wall.

The foil 2 is cut to a mainly rectangular shape as shown in fig. 4 and is provided with a lip 7 and a strip 8. In order to locate the foil 2 in the correct position around the intestine, a mainly slot-like aperture is cut out and into which the lip 7 is inserted when the foil 2 is laid around the intestine and the strip 8 is laid 8 is laid around the mesentery 6. After laying the strip 8 and in order to fix it by its end to or against the the edge 10 of the foil 2, it has been shown in practice that the length 11 of the strip 8 needs to be approximately equal to the length of the extension 12 thereof.

## Claims

1. A foil of tanned, sheep dermal-collagen characterised in that it is used as a fast working medium for uniting (a part of) living tissue to (a part of) another living tissue.

2. A foil as claimed in claim 1 characterised in that the said foil is used to fix adjacently lying outer surfaces of the wall of a transporting organ with respect to one another by the introduction of the said foil around and between both the said surfaces of the organ into which a valve has been invaginated.

3. A foil as claimed in claim 2 characterised in that the said foil is mainly rectangular in form and of which two parallel sides thereof have a length that is approximately equal to the circumference of a cross-section of the organ whilst when viewed in one direction the foil extends along one side with a lip and on the other side with a strip the length of which is approximately equal to the length of an extension of the said side.

4. A foil as claimed in claim 1 characterised in that the said foil is used to unite severed hard cerebral membranes together after an operation.

5. A foil as claimed in claim 1 characterised in that the said foil is used to unite separate parts of a transporting organ.

6. A foil as claimed in claim 1 characterised in that the said foil is used for fixing organs in their normal anatomical places.

7. A foil as claimed in claim 1 characterised in that the said foil is used as a means for uniting ruptures between parts of an abdomen wall and for filling up abdomen wall defects.

8. A foil as claimed in claim 1 characterised in that the said foil is used as a means of uniting separated tendons to one another.

9. A foil as claimed in claim 1 characterised in that the said foil is used as a means for protecting sutures.

10. A foil as claimed in claim 1 characterised in that the said foil is used as an artificial ligament for uniting joint parts to one another.

11. A foil as claimed in claim 1 characterised in that the said foil is used as a bone-cement.

12. A foil as claimed in any one of the foregoing claims characterised in that antiibiotics are added to the said foil.

13. A foil as claimed in any one of claims 1 to 11 inclusive characterised in that antiseptics are added to the said foil.

14. A foil as claimed in any one of claims 1 to 11 inclusive characterised in that blood anti-staunching media are added to the said foil.

15. A foil as claimed in any one of claims 1 to 11 inclusive characterised in that cell stimulants are added to to the said foil.

Fig. 1

Fig. 2

6

2

**Fig. 3**

9    6

10

12

11

7

8

**Fig. 4**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | NL-A-8 701 370 (STICHTING SURGICAL RESEARCH FOUNDATION) * Whole document * | 1-15 | A 61 L 25/00 A 61 L 27/00 A 61 L 31/00 |
| P,X | THE NETHERLANDS JOURNAL OF SURGERY, vol. 39, no. 3, June 1987, pages 90-94, NL; T.M. VAN GULIK et al.: "The processing of sheepskin for use as a dermal collagen graft - an experimental study" * Whole article * | 1-15 | |
| Y | US-A-4 066 083 (P.E. RIES) * Column 2, lines 16-18; column 3, lines 23-66 * | 1,8-13 | |
| D,Y | T.M. VAN GULIK: "Processed sheep dermal collagen as a biomaterial. An experimental study", thesis, University of Amsterdam, 1981, pages 232-278 * Pages 234, 246 * | 1,8-13 | |
| Y | WO-A-8 500 511 (J.C. MEDLEN) * Page 5, lines 25-35; page 31, lines 5-16 * | 8,10 | TECHNICAL FIELDS SEARCHED (Int. Cl.4) A 61 L C 08 H C 08 L |
| Y | EP-A-0 069 260 (DR. RUHLAND NACHF. GmbH) * Page 3, lines 24-26; claims * | 12,13 | |
| Y | DE-A-3 038 319 (DR. H. REIMER) * Claims * | 11 | |

The present search report has been drawn up for all claims

| Place of search | Date of completion of the search | Examiner |
|---|---|---|
| THE HAGUE | 14-10-1988 | ESPINOSA Y CARRETERO M. |

EPO FORM 1503 03.82 (P0401)